# EUROPÄISCHE PATENTANMELDUNG

(11) **EP 0 729 952 A1**
(43) Veröffentlichungstag der Anmeldung: **04.09.1996**
(21) Anmeldenummer: 96102316.5
(22) Anmeldetag: 16.02.1996
(51) Int. Cl.: C07D 319/06, C09K 19/34

(54) **1-Fluorcyclohexen-Dioxan Derivate**

(30) Priorität: 03.03.1995 CH 608/95
(71) Anmelder: F. HOFFMANN-LA ROCHE AG, 4070 Basel (CH)
(72) Erfinder: Buchecker, Richard, 8008 Zürich (CH); Marck, Guy, 68170 Rixheim (FR)
(74) Vertreter: Kjellsaa-Berger, Hanny, Dr.

(57) **Zusammenfassung**

Die vorliegende Erfindung betrifft Verbindungen der allgemeinen Formel 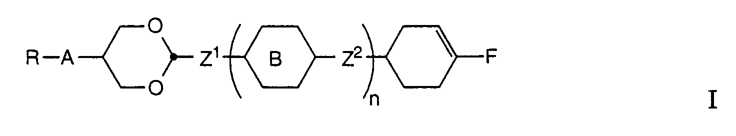 worin
- R: Alkyl oder Alkenyl mit höchstens 12 Kohlenstoffatomen;
- A: eine Einfachbindung oder trans-1,4-Cyclohexylen;
- Z¹ und Z²: eine Einfachbindung oder -CH₂CH₂-;
- Ring B: trans-1,4-Cyclohexylen oder 1,4-Phenylen bedeuten; und
- n: 0 oder 1 ist;
mit der Massgabe, dass Z¹ und Z² nicht gleichzeitig -CH₂CH₂- sein können;
sowie flüssigkristalline Gemische, die solche Verbindungen enthalten und die Verwendung solcher Verbindungen bzw. Gemische für elektro-optische Anzeigevorrichtungen.

## Beschreibung

Die vorliegende Erfindung betrifft neue flüssigkristalline Fluorcyclohexen-Dioxan-Derivate, sowie flüssigkristalline Mischungen, welche solche Verbindungen enthalten, und die Verwendung solcher Verbindungen und Mischungen in elektro-optischen Vorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und es erlauben, Mischungen mit kurzen Ansprechzeiten, tiefen Schwellenspannungen und einen hohem Kontrast herzustellen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Diese letztere Eigenschaft ist vor allem für TFT-Zellen von besonderer Wichtigkeit. Leider führen aber Komponenten mit hoher dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen.

Durch die vorliegende Erfindung werden nun Verbindungen mit einer überraschend hohen dielektrischen Anisotropie und gleichzeitig geringer Leitfähigkeit sowie einer relativ kleinen optischen Anisotropie zur Verfügung gestellt. Es sind dies die Verbindungen der allgemeinen Formel 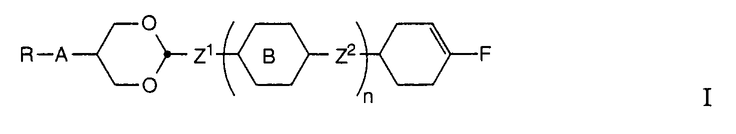 worin
- R: Alkyl oder Alkenyl mit höchstens 12 Kohlenstoffatomen;
- A: eine Einfachbindung oder trans-1,4-Cyclohexylen ;
- Z¹ und Z²: eine Einfachbindung oder -CH₂CH₂-;
- Ring B: trans-1,4-Cyclohexylen oder 1,4-Phenylen bedeuten; und
- n: 0 oder 1 ist;
mit der Massgabe, dass Z¹ und Z² nicht gleichzeitig -CH₂CH₂- sein können.

Der Ausdruck "Alkyl oder Alkenyl mit höchstens 12 Kohlenstoffatomen" bedeutet im Rahmen der vorliegenden Erfindung geradkettige oder verzweigte (gegebenenfalls chirale) Alkylgruppen oder 1E-Alkenyl-, 3E-Alkenyl- oder Alkenylgruppen mit endständiger Doppelbindung. Diese Gruppen haben höchstens 12 , vorzugsweise jedoch höchstens 7 Kohlenstoffatome. Es sind dies beispielsweise Methyl, Ethyl, Propyl, Butyl, Pentyl, Hexyl, Heptyl, Isopropyl, 2-Methyl-butyl, 2-Methyl-pentyl, 2-Methyl-hexyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 5-Hexenyl, 6-Heptenyl und dergleichen.

Bevorzugt werden die erfindungsgemässen Verbindungen der Formeln 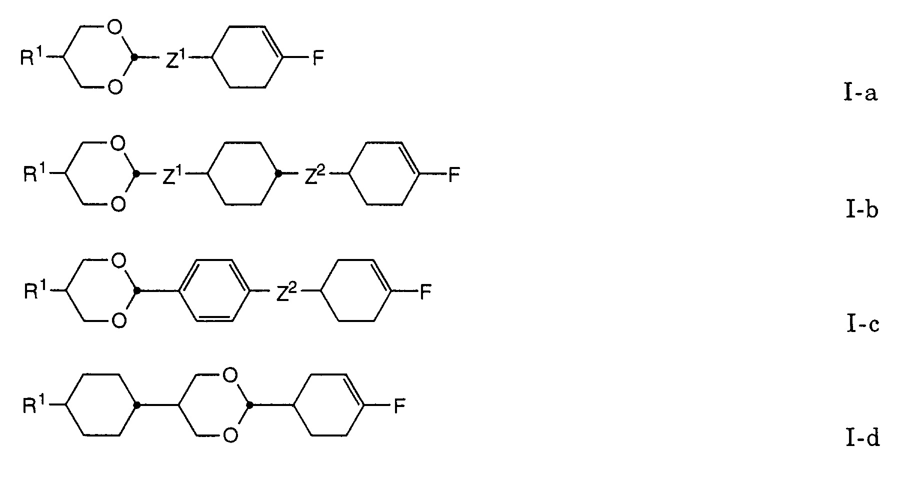 worin
- R¹: geradkettiges Alkyl oder geradkettiges 1E- oder 3E-Alkenyl mit höchstens 7 Kohlenstoffatomen bedeutet; und
- Z¹ und Z²: wie weiter oben definiert sind.

Besonders bevorzugt sind Verbindungen der Formeln I-a, I-b und I-c, insbesondere diejenigen, worin Z¹ und, wo vorhanden, auch Z² eine Einfachbindung bedeutet; dh. Verbindungen der Formeln 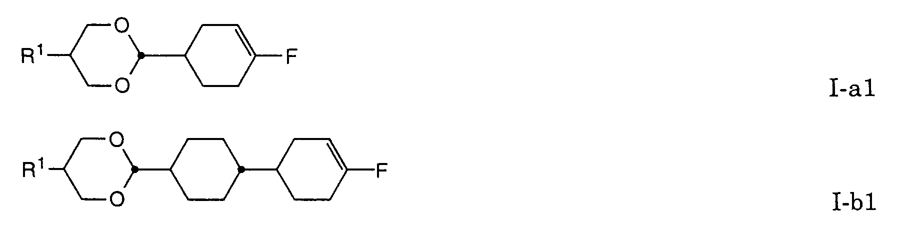 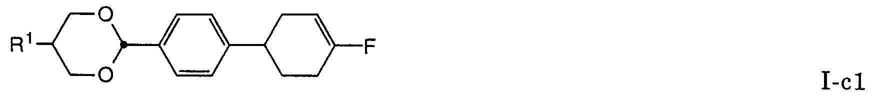 worin R¹ die obengenannte Bedeutung hat.

Ganz besonders bevorzugt werden Verbindungen der Formeln I und I-a, I-b und I-c, sowie Verbindungen der Formeln I-a1, I-b1 und I-c1, worin R¹ Ethyl, Propyl, Butyl, Pentyl, Vinyl, 1E-Propenyl, 1E-Butenyl, 1E-Pentenyl, 3-Butenyl, 3E-Pentenyl oder 4-Pentenyl bedeutet.

Die Verbindungen der allgemeinen Formel I können in an sich bekannter Weise beispielsweise nach dem im Schema 1 aufgezeichneten Weg hergestellt werden, wobei den in den Formeln angegebenen Symbolen die obenerwähnten Bedeutungen zukommen. So kann beispielsweise die Fluorierung der Ketone **1** mit Diethylaminoschwefeltrifluorid (DAST) zu den Fluorcyclohexenen **2** analog zu einem Verfahren, welches in US 4 212 815 oder in Organic Reactions 35, 531 (1988) beschrieben ist, durchgeführt werden. Die Reaktion wird vorzugsweise in polaren, aprotischen, inerten Lösungsmitteln wie Dimethoxyethan, Diglym, Dioxan und dergleichen bei Temperaturen von ca. 20°C bis ca. 50°C durchgeführt. Die Entstehung von geminalen Difluorcyclohexan-Derivaten kann beispielsweise durch Zugabe einer katalytischen Menge von Säure wie Oleum oder Trifluormethansulfonsäure unterdrückt werden. Die weiteren Reaktionsschritte, dh. die Reduktion des Esters **2** zum Aldehyd **3** und die nachfolgende Acetalisierung mit einem Diol **4**, welche zu den Dioxanen der Formel I führen, sind Standardmethoden der organischen Chemie und jedem Fachmann bekannt.

### Schema 1

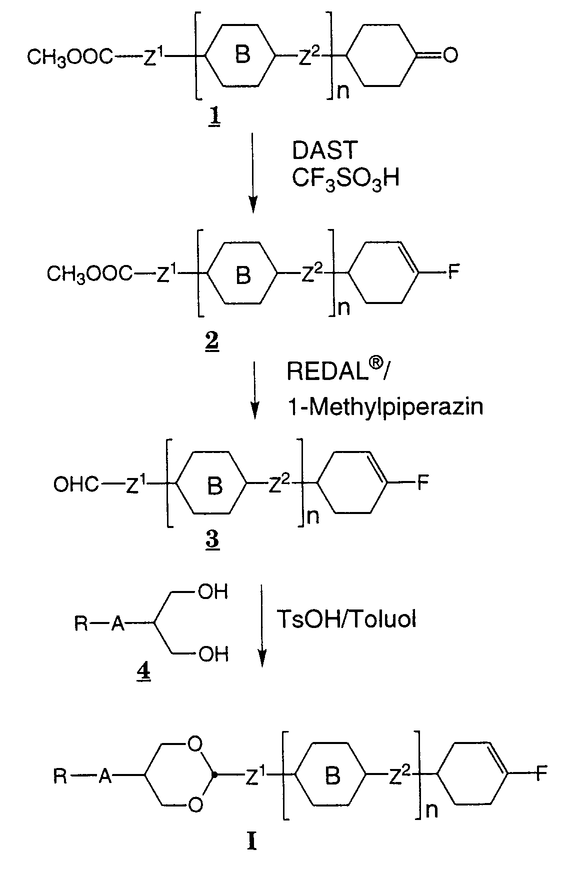

Die Ketone **1** sowie die Diole **4** sind bekannt oder Analoge bekannter Verbindungen und können nach bekannten Methoden hergestellt werden. So wird die Herstellung der Ketone **1** ausführlich in der Literatur beschrieben, beispielsweise in EP-A 122 389. Ferner werden Diole **4** generell bei der Herstellung von 1,3-Dioxanen in der Flüssigkristallchemie eingesetzt und viele davon sind beschrieben, beispielsweise in EP-A 433 836.

Die Verbindungen der Formel I können in Form von Gemischen untereinander und/oder mit anderen Flüssigkristallkomponenten verwendet werden.

Die Erfindung betrifft daher ebenfalls flüssigkristalline Gemische mit mindestens 2 Komponenten, welche dadurch gekennzeichnet sind, dass mindestens eine Komponente eine Verbindung der Formel I ist. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die erfindungsgemässen Verbindungen zeichnen sich in Gemischen durch ihre chemische Stabilität aus. Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-20 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 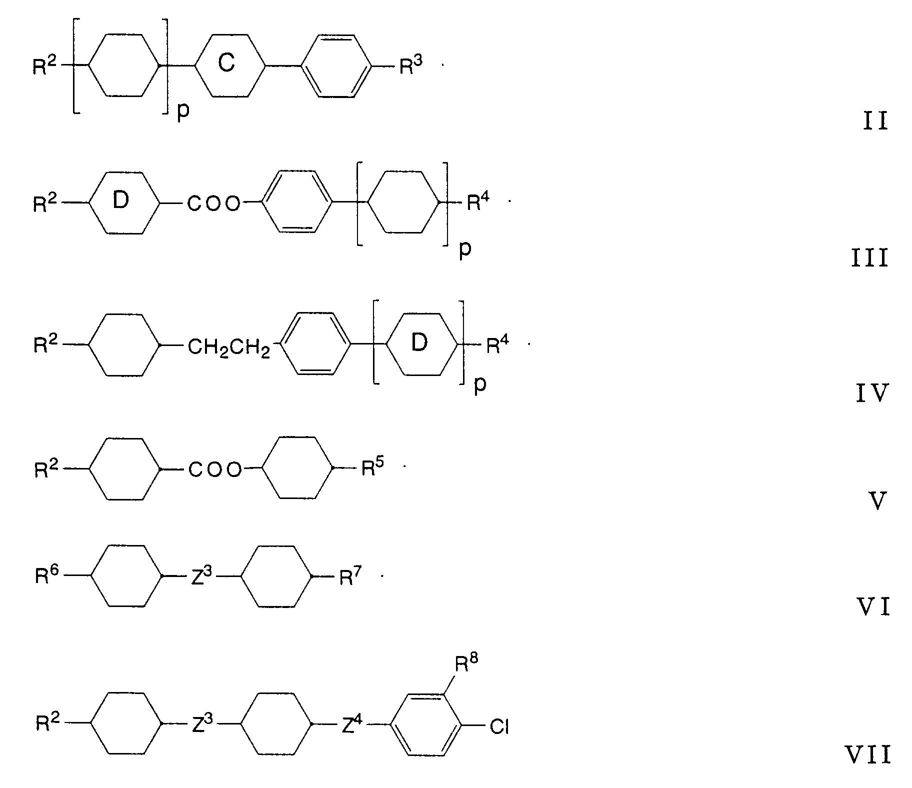 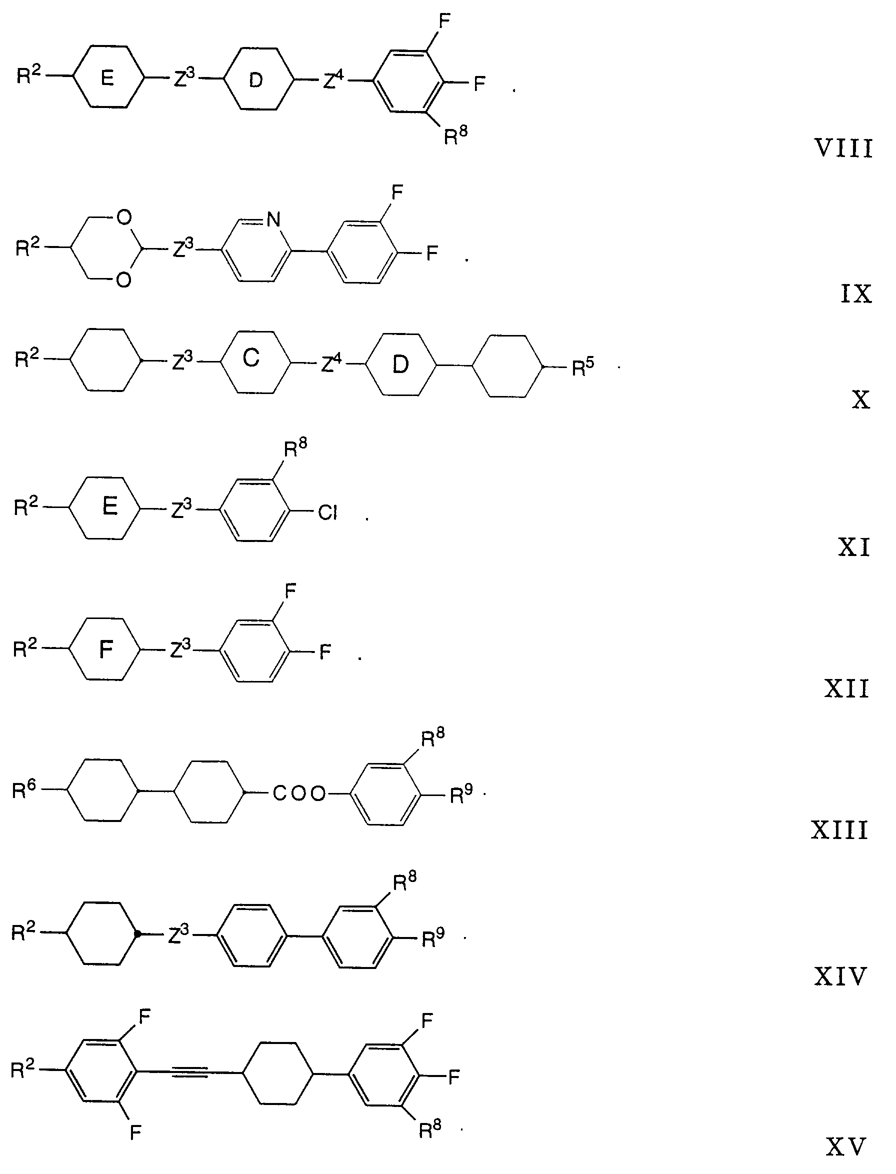 worin
- R² und R⁵: Alkyl, Alkoxyalkyl, 3E-Alkenyl, 4-Alkenyl oder an gesättigten Ringen auch 1E-Alkenyl;
- p: 0 oder 1;
- Ring C: 1,4-Phenylen, trans-1,4-Cyclohexylen oder trans-1,3-Dioxan-2,5-diyl;
- R³: Fluor, Alkyl, 3E-Alkenyl, 4-Alkenyl, Alkoxy, 2E-Alkenyloxy, 3-Alkenyloxy oder 1-Alkinyl;
- Ring D: 1,4-Phenylen oder trans-1,4-Cyclohexylen;
- R⁴: Alkyl, 3E-Alkenyl, 4-Alkenyl, oder an trans-1,4-Cyclohexylen auch 1E-Alkenyl, oder an 1,4-Phenylen auch Alkoxy, 2E-Alkenyloxy oder 3-Alkenyloxy;
- R⁶: Alkyl, 1E-Alkenyl, 3E-Alkenyl oder 4-Alkenyl;
- R⁷: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl;
- Z³ und Z⁴: eine einfache Kovalenzbindung oder -CH₂CH₂- bedeuten, wobei zwei aromatische Ringe immer durch eine einfache Kovalenzbindung verknüpft sind;
- Ring E: trans-1,4-Cyclohexylen oder trans-1,3-Dioxan-2,5-diyl;
- R⁸: Wasserstoff oder Fluor ;
- R⁹: Fluor oder Chlor; und
- Ring F: trans-1,4-Cyclohexylen oder trans-1,3-Dioxan-2,5-diyl bedeuten.

Die im Zusammenhang mit den Verbindungen der Formeln II bis XV verwendeten Ausdrücke werden im folgenden erläutert.
"Aromatische Ringe" bezeichnet Ringe, wie beispielsweise 1,4-Phenylen, Pyridin-2,5-diyl oder Pyrimidin-2,5-diyl.
"Gesättigte Ringe" bezeichnet trans-1,4-Cyclohexylen oder trans-1,3-Dioxan-2,5-diyl.
"Alkyl" bedeutet vorzugsweise geradkettige Gruppen mit 1 bis 12 Kohlenstoffatomen, besonders bevorzugte Reste haben 1 bis 7 Kohlenstoffatomen, wie beispielsweise Methyl, Ethyl, Propyl, Butyl, Pentyl, Hexyl oder Heptyl.
"Alkyloxyalkyl" bedeutet vorzugsweise geradkettige Reste, wie beispielsweise Methoxymethyl, Ethoxymethyl, Propyloxymethyl, Butyloxymethyl und dergleichen.
"Alkoxy" bedeutet vorzugsweise geradkettige Reste, wie beispielsweise Methoxy, Ethoxy, Propyloxy, Butyloxy, Pentyloxy, Hexyloxy, Heptyloxy und dergleichen.
"1E-Alkenyl" bedeutet vorzugsweise geradkettige Alkenylreste, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.
"3E-Alkenyl" bedeutet vorzugsweise geradkettige Alkenylreste, in denen die Doppelbindung sich in 3-Stellung befindet, wie beispielsweise 3-Butenyl, 3E-Pentenyl, 3E-Hexenyl, 3E-Heptenyl und dergleichen.
"4-Alkenyl" bedeutet vorzugsweise geradkettige Alkenylreste, in denen die Doppelbindung sich in 4-Stellung befindet, wie beispielsweise 4-Pentenyl, 4-Hexenyl, 4-Heptenyl und dergleichen.
"2E- bzw. 3-Alkenyloxy" bedeutet in diesem Zusammenhang vorzugsweise geradkettige Alkenyloxyreste, in denen die Doppelbindung sich in 2- bzw. 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.
"1-Alkinyl" bedeutet in diesem Zusammenhang vorzugsweise geradkettige Alkinylreste, in denen die Dreifachbindung sich in 1-Stellung befindet, wie beispielsweise Ethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe der Helix, Farbe, Extinktion und dergleichen bestimmt. Im allgemeinen beträgt der Anteil optisch aktiver Verbindungen und dichroitischer Farbstoffe höchstens je etwa 10 Gew.% im Gesamtgemisch.

Die Herstellung der flüssigkristallinen Gemische und der elektrooptischen Vorrichtungen kann in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische Phase, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn die optische Anisotropie.

### Beispiel 1

a) Unter Stickstoffbegasung wurden 8,9 ml Natrium-bis(2-methoxyethoxy)-aluminium-hydrid und 9 ml Toluol auf 0°C gekühlt und innert 30 Minuten mit einer Lösung von 4,15 ml 1-Methylpiperazin in 20 ml Toluol versetzt. Diese Lösung wurde noch 30 Minuten bei 0°C gerührt und anschliessend innert 45 Minuten zu einer auf -50°C gekühlten Lösung von 3,0 g Methyl trans-4-(4-Fluoro-cyclohex-3-enyl)cyclohexancarboxylat in 40 ml Toluol getropft. Nach 2 Stunden Rühren bei -50°C wurde das Reaktionsgemisch mit 20 ml Ethanol versetzt und auf eine 10%ige Natriumtartrat-Lösung gegossen. Die organische Phase wurde zweimal mit Wasser extrahiert. Die wässrige Phase je zweimal mit Ether extrahiert und die organischen Phasen vereinigt, über Magnesiumsulfat getrocknet, filtriert, und das Filtrat eingedampft. Der Rückstand wurde an Kieselgel (5% Ethylacetat/Cyclohexan) gereinigt und ergab 1,77 g trans-4-(4-Fluoro-cyclohex-3-enyl)cyclohexancarbaldehyd.
b) In einer mit Stickstoff begasten Apparatur mit Wasserabscheider werden 500 mg trans-4-(4-Fluoro-cyclohex-3-enyl)cyclohexancarbaldehyd, 310 mg 2-Propyl-propan-1,3-diol und 50 mg p-Toluolsulfonsäure in 8 ml Toluol 1 Stunde am Rückfluss gekocht. Nach dem Abkühlen wird die Reaktionslösung auf Wasser/Ether verteilt. Die organische Phase wurde zweimal mit Wasser gewaschen. Die wässrigen Phasen wurden je zweimal mit Ether extrahiert. Die organischen Phasen wurden vereinigt, über Magnesiumsulfat getrocknet, filtriert und das Filtrat eingedampft. Das Rohprodukt wurde mehrmals aus Ethanol/1% Ethylacetat umkristallisiert und ergab 350 mg trans-2-[trans-4-(4-Fluoro-cyclohex-3-enyl)cyclohexyl]-5-propyl-[1,3]-dioxan, Smp. (C-N) < Raumtemperatur; S-N 104°C, Klp. (N-I) 118,3°C;
   Auf analoge Weise können folgende Verbindungen hergestellt werden:
   trans-2-[trans-4-(4-Fluoro-cyclohex-3-enyl)cyclohexyl]-5-ethyl-[1,3]-dioxan,
   trans-2-[trans-4-(4-Fluoro-cyclohex-3-enyl)cyclohexyl]-5-butyl-[1,3]-dioxan,
   trans-2-[trans-4-(4-Fluoro-cyclohex-3-enyl)cyclohexyl]-5-pentyl-[1,3]-dioxan,
   trans-2-[trans-4-(4-Fluoro-cyclohex-3-enyl)cyclohexyl]-5-vinyl-[1,3]-dioxan,
   trans-2-[trans-4-(4-Fluoro-cyclohex-3-enyl)cyclohexyl]-5-(prop-1E-enyl)-[1,3]-dioxan,
   trans-2-[trans-4-(4-Fluoro-cyclohex-3-enyl)cyclohexyl]-5-(but-1E-enyl)-[1,3]-dioxan,
   trans-2-[trans-4-(4-Fluoro-cyclohex-3-enyl)cyclohexyl]-5-(pent-1E-enyl)-[1,3]-dioxan,
   trans-2-[trans-4-(4-Fluoro-cyclohex-3-enyl)cyclohexyl]-5-(but-3-enyl)-[1,3]-dioxan,
   trans-2-[trans-4-(4-Fluoro-cyclohex-3-enyl)cyclohexyl]-5-(pent-3E-enyl)-[1,3]-dioxan,
   trans-2-[4-(4-Fluoro-cyclohex-3-enyl)phenyl]-5-ethyl-[1,3]-dioxan,
   trans-2-[4-(4-Fluoro-cyclohex-3-enyl)phenyl]-5-propyl-[1,3]-dioxan,
   trans-2-[4-(4-Fluoro-cyclohex-3-enyl)phenyl]-5-butyl-[1,3]-dioxan,
   trans-2-[4-(4-Fluoro-cyclohex-3-enyl)phenyl]-5-pentyl-[1,3]-dioxan,
   trans-2-[4-(4-Fluoro-cyclohex-3-enyl)phenyl]-5-vinyl-[1,3]-dioxan,
   trans-2-[4-(4-Fluoro-cyclohex-3-enyl)phenyl]-5-(prop-1E-enyl)-[1,3]-dioxan,
   trans-2-[4-(4-Fluoro-cyclohex-3-enyl)phenyl]-5-(but-1E-enyl)-[1,3]-dioxan,
   trans-2-[4-(4-Fluoro-cyclohex-3-enyl)phenyl]-5-(pent-1E-enyl)-[1,3]-dioxan,
   trans-2-[4-(4-Fluoro-cyclohex-3-enyl)phenyl]-5-(but-3-enyl)-[1,3]-dioxan,
   trans-2-[4-(4-Fluoro-cyclohex-3-enyl)phenyl]-5-(pent-3E-enyl)-[1,3]-dioxan,
   trans-2-(4-Fluoro-cyclohex-3-enyl)-5-ethyl-[1,3]-dioxan,
   trans-2-(4-Fluoro-cyclohex-3-enyl)-5-propyl-[1,3]-dioxan,
   trans-2-(4-Fluoro-cyclohex-3-enyl)-5-butyl-[1,3]-dioxan,
   trans-2-(4-Fluoro-cyclohex-3-enyl)-5-pentyl-[1,3]-dioxan,
   trans-2-(4-Fluoro-cyclohex-3-enyl)-5-vinyl-[1,3]-dioxan,
   trans-2-(4-Fluoro-cyclohex-3-enyl)-5-(prop-1E-enyl)-[1,3]-dioxan,
   trans-2-(4-Fluoro-cyclohex-3-enyl)-5-(but-1E-enyl)-[1,3]-dioxan,
   trans-2-(4-Fluoro-cyclohex-3-enyl)-5-(pent-1E-enyl)-[1,3]-dioxan,
   trans-2-(4-Fluoro-cyclohex-3-enyl)-5-(but-3-enyl)-[1,3]-dioxan,
   trans-2-(4-Fluoro-cyclohex-3-enyl)-5-(pent-3E-enyl)-[1,3]-dioxan,
   trans-2-(4-Fluoro-cyclohex-3-enyl)-5-(trans-4-ethyl-cyclohexyl)-[1,3]-dioxan,
   trans-2-(4-Fluoro-cyclohex-3-enyl)-5-(trans-4-propyl-cyclohexyl)-[1,3]-dioxan,
   trans-2-(4-Fluoro-cyclohex-3-enyl)-5-(trans-4-butyl-cyclohexyl)-[1,3]-dioxan,
   trans-2-(4-Fluoro-cyclohex-3-enyl)-5-(trans-4-pentyl-cyclohexyl)-[1,3]-dioxan,
   trans-2-(4-Fluoro-cyclohex-3-enyl)-5-(trans-4-vinyl-cyclohexyl)-[1,3]-dioxan,
   trans-2-(4-Fluoro-cyclohex-3-enyl)-5-[trans-4-(prop-1E-enyl)-cyclohexyl]-[1,3]-dioxan,
   trans-2-(4-Fluoro-cyclohex-3-enyl)-5-[trans-4-(but-1E-enyl)-cyclohexyl]-[1,3]-dioxan,
   trans-2-(4-Fluoro-cyclohex-3-enyl)-5-[trans-4-(pent-1E-enyl)-cyclohexyl]-[1,3]-dioxan,
   trans-2-(4-Fluoro-cyclohex-3-enyl)-5-[trans-4-(but-3-enyl)-cyclohexyl]-[1,3]-dioxan,
   trans-2-(4-Fluoro-cyclohex-3-enyl)-5-[trans-4-(pent-3E-enyl)-cyclohexyl]-[1,3]-dioxan,
   trans-2-[2-(4-Fluoro-cyclohex-3-enyl)ethyl]-5-ethyl-[1,3]-dioxan,
   trans-2-[2-(4-Fluoro-cyclohex-3-enyl)ethyl]-5-propyl-[1,3]-dioxan,
   trans-2-[2-(4-Fluoro-cyclohex-3-enyl)ethyl]-5-butyl-[1,3]-dioxan,
   trans-2-[2-(4-Fluoro-cyclohex-3-enyl)ethyl]-5-pentyl-[1,3]-dioxan,
   trans-2-[2-(4-Fluoro-cyclohex-3-enyl)ethyl]-5-vinyl-[1,3]-dioxan,
   trans-2-[2-(4-Fluoro-cyclohex-3-enyl)ethyl]-5-(prop-1E-enyl)-[1,3]-dioxan,
   trans-2-[2-(4-Fluoro-cyclohex-3-enyl)ethyl]-5-(but-1E-enyl)-[1,3]-dioxan,
   trans-2-[2-(4-Fluoro-cyclohex-3-enyl)ethyl]-5-(pent-1E-enyl)-[1,3]-dioxan,
   trans-2-[2-(4-Fluoro-cyclohex-3-enyl)ethyl]-5-(but-3-enyl)-[1,3]-dioxan,
   trans-2-[2-(4-Fluoro-cyclohex-3-enyl)ethyl]-5-(pent-3E-enyl)-[1,3]-dioxan,
   trans-2-(2-[trans-4-(4-Fluoro-cyclohex-3-enyl)cyclohexyl]ethyl]-5-ethyl-[1,3]-dioxan,
   trans-2-{2-[trans-4-(4-Fluoro-cyclohex-3-enyl)cyclohexyl]ethyl}-5-propyl-[1,3]-dioxan,
   trans-2-{2-[trans-4-(4-Fluoro-cyclohex-3-enyl)cyclohexyl]ethyl}-5-butyl-[1,3]-dioxan,
   trans-2-{2-[trans-4-(4-Fluoro-cyclohex-3-enyl)cyclohexyl]ethyl}-5-pentyl-[1,3]-dioxan,
   trans-2-{2-[trans-4-(4-Fluoro-cyclohex-3-enyl)cyclohexyl]ethyl}-5-vinyl-[1,3]-dioxan,
   trans-2-{2-[trans-4-(4-Fluoro-cyclohex-3-enyl)cyclohexyl]ethyl}-5-(prop-1E-enyl)-[1,3]-dioxan,
   trans-2-{2-[trans-4-(4-Fluoro-cyclohex-3-enyl)cyclohexyl]ethyl}-5-(but-1E-enyl)-[1,3]-dioxan,
   trans-2-{2-[trans-4-(4-Fluoro-cyclohex-3-enyl)cyclohexyl]ethyl}-5-(pent-1E-enyl)-[1,3]-dioxan,
   trans-2-{2-[trans-4-(4-Fluoro-cyclohex-3-enyl)cyclohexyl]ethyl}-5-(but-3-enyl)-[1,3]-dioxan,
   trans-2-{2-[trans-4-(4-Fluoro-cyclohex-3-enyl)cyclohexyl]ethyl}-5-(pent-3E-enyl)-[1,3]-dioxan,
   trans-2-{4-[2-(4-Fluoro-cyclohex-3-enyl)ethyl]phenyl}-5-ethyl-[1,3]-dioxan,
   trans-2-{4-[2-(4-Fluoro-cyclohex-3-enyl)ethyl]phenyl}-5-propyl-[1,3]-dioxan,
   trans-2-{4-[2-(4-Fluoro-cyclohex-3-enyl)ethyl]phenyl}-5-butyl-[1,3]-dioxan,
   trans-2-{4-[2-(4-Fluoro-cyclohex-3-enyl)ethyl]phenyl}-5-pentyl-[1,3]-dioxan,
   trans-2-{4-[2-(4-Fluoro-cyclohex-3-enyl)ethyl]phenyl}-5-vinyl-[1,3]-dioxan,
   trans-2-{4-[2-(4-Fluoro-cyclohex-3-enyl)ethyl]phenyl}-5-(prop-1E-enyl)-[1,3]-dioxan,
   trans-2-{4-[2-(4-Fluoro-cyclohex-3-enyl)ethyl]phenyl}-5-(but-1E-enyl)-[1,3]-dioxan,
   trans-2-{4-[2-(4-Fluoro-cyclohex-3-enyl)ethyl]phenyl}-5-(pent-1E-enyl)-[1,3]-dioxan,
   trans-2-{4-[2-(4-Fluoro-cyclohex-3-enyl)ethyl]phenyl}-5-(but-3-enyl)-[1,3]-dioxan,
   trans-2-{4-[2-(4-Fluoro-cyclohex-3-enyl)ethyl]phenyl}-5-(pent-3E-enyl)-[1,3]-dioxan,
   trans-2-{4-[2-(4-Fluoro-cyclohex-3-enyl)ethyl]cyclohexyl}-5-ethyl-[1,3]-dioxan,
   trans-2-{4-[2-(4-Fluoro-cyclohex-3-enyl)ethyl]cyclohexyl}-5-propyl-[1,3]-dioxan,
   trans-2-{4-[2-(4-Fluoro-cyclohex-3-enyl)ethyl]cyclohexyl}-5-pentyl-[1,3]-dioxan,
   trans-2-{4-[2-(4-Fluoro-cyclohex-3-enyl)ethyl]cyclohexyl}-5-(prop-1E-enyl)-[1,3]-dioxan.

### Beispiel 2

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 µm Plattenabstand bei 22°C gemessen; als Betriebsspannung wurde der 2,5-fache Wert der Schwellenspannung (V₁₀) gewählt. Die entsprechenden Daten für 4-(trans-4-Pentyl-cyclohexyl)benzonitril betragen: Klp. (N-I) = 54.6°C, V₁₀ = 1,62 V, tₒₙ = 22ms, t_{off} = 42 ms, Δn = 0,120.

### BM-1

- 90 Gew.%: 4-(trans-4-Pentyl-cyclohexyl)benzonitril
- 10 Gew.%: trans-2-[trans-4-(4-Fluoro-cyclohex-3-enyl)cyclohexyl]-5-propyl-[1,3]-dioxan
Klp. (N/I): 55.9°C, V₁₀ = 1,54 V, tₒₙ = 29 ms, t_{off} = 47 ms, Δn = 0,114

### BM-2

- 80 Gew.%: 4-(trans-4-Pentyl-cyclohexyl)benzonitril
- 20 Gew.%: trans-2-[trans-4-(4-Fluoro-cyclohex-3-enyl)cyclohexyl]-5-propyl-[1,3]-dioxan
Klp. (N/I): 58.2°C, V₁₀ = 1,49 V, tₒₙ = 34 ms, t_{off} = 55 ms, Δn = 0,107

## Patentansprüche

1. Verbindungen der allgemeinen Formel 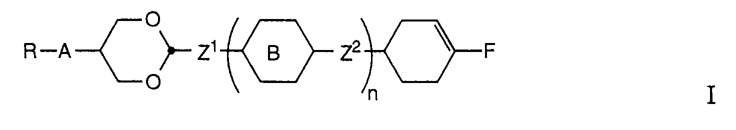 worin
R Alkyl oder Alkenyl mit höchstens 12 Kohlenstoffatomen;
A eine Einfachbindung oder trans-1,4-Cyclohexylen ;
Z¹ und Z² eine Einfachbindung oder -CH₂CH₂-;
Ring B trans-1,4-Cyclohexylen oder 1,4-Phenylen bedeuten; und
n 0 oder 1 ist;
mit der Massgabe, dass Z¹ und Z² nicht gleichzeitig -CH₂CH₂- sein können.

2. Verbindungen gemäss Anspruch 1 der Formeln 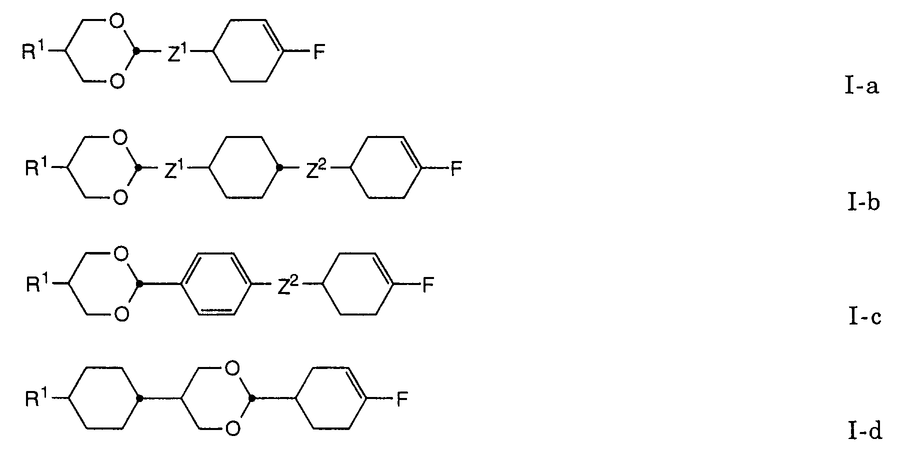 worin
R¹ geradkettiges Alkyl oder geradkettiges 1E- oder 3E-Alkenyl mit höchstens 7 Kohlenstoffatomen; und
Z¹ und Z² eine Einfachbindung oder -CH₂CH₂- bedeuten.

3. Verbindungen nach einem der Ansprüche 1 oder 2, dadurch gekennzeichnet, dass Z¹ und Z² eine Einfachbindung bedeuten.

4. Verbindungen nach einem der Ansprüche 2 oder 3 der Formeln 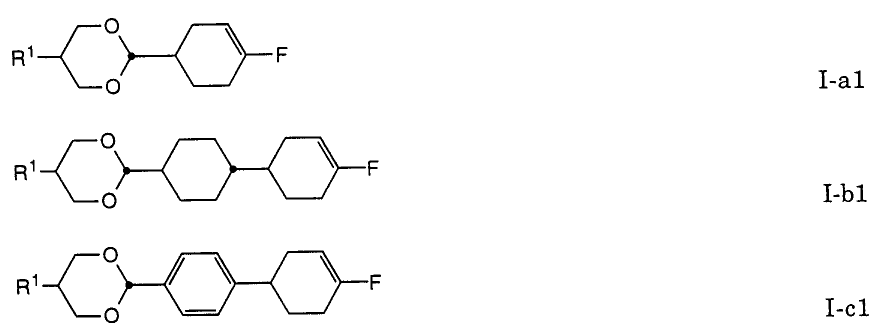 worin
R¹ geradkettiges Alkyl oder geradkettiges 1E- oder 3E-Alkenyl mit höchstens 7 Kohlenstoffatomen bedeutet.

5. Verbindungen nach einem der Ansprüche 1 bis 4, dadurch gekennzeichnet, dass R¹ Ethyl, Propyl, Butyl, Pentyl, Vinyl, 1E-Propenyl, 1E-Butenyl, 1E-Pentenyl, 3-Butenyl, 3E-Pentenyl oder 4-Pentenyl bedeutet.

6. Flüssigkristallines Gemisch mit mindestens 2 Komponenten, dadurch gekennzeichnet, dass mindestens eine Komponente eine Verbindung der in Anspruch 1 definierten Formel I ist.

7. Flüssigkristallines Gemisch nach Anspruch 6, dadurch gekennzeichnet, dass der Anteil an Verbindungen der Formel I 1-70 Gew.-% beträgt.

8. Flüssigkristallines Gemisch nach Anspruch 6 oder 7, dadurch gekennzeichnet, dass der Anteil an Verbindungen der Formel I 5-20 Gew.-% beträgt.

9. Verwendung der Verbindungen der in Anspruch 1 definierten Formel I für elektro-optische Vorrichtungen.

10. Verwendung von flüssigkristallinen Mischungen nach einem der Ansprüche 6 bis 8 für elektro-optische Vorrichtungen.
